# EUROPEAN PATENT APPLICATION

(11) **EP 3 550 028 A1**
(43) Date of publication of application: **09.10.2019**
(21) Application number: 17876392.6
(22) Date of filing: 29.09.2017
(51) Int. Cl.: C12P 21/02, A01H 5/00, C07K 14/415, C12N 9/24, C12N 15/09

(54) **METHOD FOR PRODUCING USEFUL PROTEIN BY MEANS OF PLANT**

(30) Priority: 30.11.2016 JP 2016233319
(71) Applicant: Kirin Company, Limited, Tokyo 164-0001 (JP)
(72) Inventor: OHARA, Kazuaki, Tokyo 164-0001 (JP); OKAWA, Hiroshi, Tokyo 164-0001 (JP)
(74) Representative: ABG Intellectual Property Law, S.L.
(86) International application number: PCT/JP2017/035383
(87) International publication number: WO 2018/100866

(57) **Abstract**

The present invention relates to a method for producing a useful protein by a plant, including culturing a plant body capable of producing a useful protein in a container while keeping at least a part of the plant body immersed in a liquid medium to allow the plant body to grow. According to the present invention, a useful protein can be stably and efficiently produced by using a plant.

## Description

### Reference to Related Application

The present application is based on the preceding Japanese Patent Application No. 2016-233319 (filed with the Japan Patent Office on November 30, 2016) and claims the benefit of the priority, and the entire disclosure content of which is incorporated herein by reference.

### Technical Field

The present invention relates to a method for producing a substance by means of a plant. That is, the present invention relates to a method for producing a useful protein stably and efficiently by continuously culturing a plant in a state in which the plant is immersed in a liquid.

### Background Art

With the progress of plant biotechnology, production of various kinds of useful substances by utilizing the functions of primary metabolism and secondary metabolism of a plant, and the examination are actively conducted.

Conventionally, a useful substance such as a useful protein has been produced by utilizing a technique such as the separation and purification from a biological material such as microorganisms, the large-scale culture of microorganisms using a large-sized fermenter, or the large-scale culture of animal cells, a gene recombination technique, or the like. However, these techniques still have problems of the requirement for a large amount of plant investment, and further the high running cost, and the risk of contamination by an animal-derived pathogen, an E. coli-derived toxin and the like.

According to the production using a plant, there is no risk of contamination by an animal-derived pathogen, an E. coli-derived toxin and the like, and there are many cases where the safety is guaranteed from the conventional food experience. Further, if a plant that has been conventionally used for food is used, the plant itself or the part thereof such as a fruit, in which a useful protein has been accumulated, can be directly ingested without separating and purifying the produced useful protein. For this reason, the expectation for the use of a plant in production of such a substance has been increased.

As a method for producing such a substance by a plant body, cultivation in a field or a greenhouse, or solid medium culture in a room is generally known (for example, WO 2015/156340 (Patent Document 1)). For the cultivation in a field or a greenhouse, it is required to prepare a large amount of seeds and seedlings, and it is also required to move the pot of seedlings and to repot the seedlings depending on the growth stage. Further, in a case where the plant body is a genetically modified organism or the transient expression by Agrobacterium is used, it is required to have a facility for which measures to prevent the diffusion into an environment such as an isolated field or a closed-system greenhouse have been taken and to take a lot of trouble. In addition, in a case of solid medium culture in a room, it is generally required to perform the passage work by hand, and therefore, there is a problem that a large amount of labor cost is required for the growth of a plant body in order to produce a large amount of useful substances.

Further, the technique for producing a useful substance by utilizing plant cell culture has also been studied from the past.

For example, in JP 2008-525454 W (Patent Document 2), a system has been proposed in which plant cells that have been genetically modified so as to produce antibodies are grown by suspension culture to produce antibodies. However, in a plant, it is known to specifically produce and accumulate a useful substance in differentiated tissues and organs. It is known that, for example, in a secondary metabolite, berberine of Coptis is accumulated specifically in the rhizome and shikonin of Lithospermum is accumulated specifically in the root, and in a case of a protein, the protein is accumulated in seeds as a storage protein, and the like. Therefore, in a case where dedifferentiated cultured cells are used, there is a problem that the production efficiency of the useful protein is inferior to that in a case of plant tissues.

Further, it has also been conventionally attempted to produce a useful substance by producing and growing the hairy root in a plant by utilizing Agrobacterium rhizogenes (or Rhizobium rhizogenes) (Journal of the Agricultural Chemical Society of Japan, Vol. 65 (1991), No. 7, P. 1127-1128 (Non Patent Document 1)). This method is said to be suitable for the production of a useful substance produced specifically in the root, however, it is not versatile and takes a lot of trouble, and therefore, there have been almost no known practical cases.

In addition, recently, it has been reported about the method for producing a useful substance by using "temporary liquid immersion culture system" (JP 2013-504317 W (Patent Document 3)). The "temporary liquid immersion culture system" has been reported in detail, for example, in the Document by H. Etienne, et al. (Plant cell, Tissue and Organ Culture 69, pp. 215-231, 2002 (Non Patent Document 2)), and has been widely used as a method for growing plant seedlings. However, in a case where by using such a temporary liquid immersion culture system, plants are grown and the useful protein is mass-produced, a facility such as a large number of special culture containers and the labor are required, and it is not a highly versatile means.

In addition, as a technique for large-scale growth by liquid culture of a plant body, in the past, there have been only a few reports of application examples in the field of seedling of seed tuber production in potato, and the like (JP 2904924 B1 (Patent Document 4)), and to the best of the knowledge of the present inventors, et al., there is no known case where this technique is applied further to the production of a useful substance.

As described above, it has been continuously awaited to provide a means by which to achieve the improvement of the production efficiency of a useful protein by a plant.

### Related Document List

### Patent Documents

Patent Document 1: WO 2015/156340
Patent Document 2: JP 2008-515454 A (or WO 2006/040764)
Patent Document 3: JP 2013-504317 A (or WO 2011/030083)
Patent Document 4: JP 2904924 B1

### Non Patent Documents

Non Patent Document 1: Journal of the Agricultural Chemical Society of Japan, Vol. 65 (1991), No. 7, P. 1127-1128
Non Patent Document 2: Plant cell, Tissue and Organ Culture 69, pp. 215-231, 2002

### Summary of Invention

The present inventors, et al. recently have found that by culturing a plant body while keeping the plant body immersed in a liquid, the amount of the useful protein produced per unit container space is increased and the production can be efficiently performed. Further, at this time, it has been found that by performing a series of processes under the liquid immersion culture conditions, the ratio of the amount of the desired proteins to the total amount of proteins in a grown plant body is unexpectedly increased. As described above, by performing continuous culture of the series of processes under the liquid immersion culture conditions, more efficient production of a useful protein has been succeeded. For this reason, the repotting of seedlings and moving of a pot of seedlings as performed in greenhouse cultivation are not required, and the overall work has been simplified and the work efficiency can be improved. Further, by accelerating the growth rate of a plant body by the liquid immersion culture, the time production efficiency can also be improved.

In addition, in a case where a genetically modified plant body is used for producing a useful substance, it is required to confine the plant body in a closed system by regulation unless specifically authorized. According to the technique of the present inventors, et al., a series of processes can be achieved only under the liquid immersion culture conditions, and therefore, the production of a substance by the culture in a closed system or semi-closed system can be simply and easily performed. For example, in a case where a culture tank such as a bag-type culture tank that can be used by the technique of the present inventors, et al. is used, the individual bags have the closed systems, respectively, and therefore, it is further simple and easy to confine the genetically modified plant body.

The present invention is based on these findings.

Accordingly, an object of the present invention is to provide a method for stably and efficiently producing a desired protein that is a useful substance by using a plant under the liquid culture conditions.

That is, according to the present invention, the following invention is provided.
<1> A method for producing a useful protein by a plant, including culturing a plant body capable of producing a useful protein in a container while keeping at least a part of the plant body immersed in a liquid medium to allow the plant body to grow.
<2> The method described in <1> above, in which the plant body is a plant body modified so as to be able to produce a useful protein.
<3> The method described in <1> or <2> above, in which the plant body is a stable genetically modified plant.
<4> The method described in any one of <1> to <3> above, in which the useful protein to be produced is a protein for medical use or a protein for industrial use.
<5> The method described in any one of <1> to <4> above, in which the protein is a protein expressed by using a constitutive expression promoter.
<6> The method described in any one of <1> to <5> above, further including a step of extracting or separating a useful protein from a grown plant body.
<7> The method described in any one of <1> to <6> above, in which a plant species to be used is any one selected from the group consisting of potato, lettuce, tobacco, tomato, rice, barley, wheat, soybean, and maize.
<8> The method described in any one of <1> to <7> above, in which culture and growth of the plant body are performed in a sterilized container.
<9> The method described in any one of <1> to <8> above, in which the container is a bag-type culture tank.
<10> The method described in any one of <1> to <9> above, in which a large amount of plants are cultured by using a plurality of containers to mass-produce the useful protein.

### Advantageous Effects of Invention

According to the present invention, by using a plant, the desired useful protein can be efficiently produced. That is, according to the present invention, as compared with the greenhouse cultivation, the solid medium culture, or the like, the production efficiency of the desired protein can be improved by combining the viewpoints of required resources, labor, time, and the like. In other words, according to the present invention, by culturing a plant body while keeping the plant body immersed in a liquid, the amount of the useful protein to be produced per unit container space can be improved. As a result, according to the present invention, the reduction of the production cost can be achieved. Further, according to the method of the present invention, by performing a series of processes under the liquid immersion culture conditions, the ratio of the content of the desired protein to the total mass of the protein in a plant body can be significantly increased. As a result, according to the present invention, the reduction of the extraction cost can be expected, and the production of the useful protein can be performed even more efficiently. For this reason, it can be said that the method according to the present invention is a protein production method useful for the actual production of a desired protein.

In addition, according to the present invention, the repotting of seedlings and moving of a pot of seedlings as performed in greenhouse cultivation are not required, and the overall work has been simplified and the work efficiency can be improved. Further, by accelerating the growth rate of a plant body by the liquid immersion culture, the time production efficiency can also be improved.

From these things, according to the present invention, the production of a desired substance can be performed more efficiently at a low cost and in safety. In addition, a genetically modified organism is required to be confined in a closed system by regulation, however, in a case where a container such as a bag-type culture tank is used, the individual containers can be made to have the closed systems, respectively, and therefore, the containment of the genetically modified organism is easily performed, and the work can be performed even more easily.

Further, if the expression/accumulation site can be limited by selecting an appropriate promoter, the separation and purification of the produced useful protein are also performed relatively easily in many cases. In addition, if a plant part that has been conventionally used for food is used, the plant itself or the part thereof, in which a useful protein has been accumulated, can be directly ingested without performing the separation and purification. According to the present invention, it can be said that these points can be easily realized.

### Brief Description of Drawings

Fig. 1 shows the results of the experiment conducted in Example 1. Specifically, the comparison of the growth amounts of the plant bodies (potato) by the difference in the culture method is shown.
Fig. 2 shows the results of the experiment conducted in Example 2. Specifically, the comparison of the growth amounts of the plant bodies (potato) by the difference in the amount of a liquid medium is shown.
Fig. 3 shows the results of the experiment conducted in Example 2. Specifically, the amounts of the GUS produced per bag-type culture tank are shown.
Fig. 4 shows the results of the experiment conducted in Example 3. Specifically, the GUS activities (leaf) of the gas phase part and liquid phase part of a bag-type culture tank are shown.
Fig. 5 shows the results of the experiment conducted in Example 3. Specifically, the GUS activities (stem) of the gas phase part and liquid phase part of a bag-type culture tank are shown.
Fig. 6 shows the results of the experiment conducted in Example 4. Specifically, the GUS activities (leaf) of the liquid immersion culture and the soil cultivation are shown.
Fig. 7 shows the results of the experiment conducted in Example 5. Specifically, the comparison of the growth amounts of the plant bodies (lettuce) in the same container by the difference in the culture method is shown.
Fig. 8 shows the results of the experiment conducted in Example 5. Specifically, the comparison of the GUS activities by the difference in the culture method is shown.
Fig. 9 shows the results of the experiment conducted in Example 5. Specifically, the amounts of the GUS produced per the same container (4 weeks) are shown.
Fig. 10 shows the results of the experiment conducted in Example 5. Specifically, the comparison of the contents of transferrin by the difference in the culture method is shown.
Fig. 11 shows the results of the experiment conducted in Example 5. Specifically, the amounts of the transferrin produced per the same container (4 weeks) are shown.
Fig. 12 shows an example of the single container (bag-type culture tank) in the use state.
Fig. 13 shows an example of the state in which a useful protein is mass-produced by using culture bags (bag-type culture tanks).

### Description of Embodiments

Hereinafter, the embodiment of the present invention will be described.

As described above, the present invention relates to a method for producing a useful protein by a plant, including culturing a plant body capable of producing a useful protein in a container while keeping at least a part of the plant body immersed in a liquid to allow the plant body to grow.

According to a preferred embodiment of the present invention, this method is performed under the liquid culture conditions.

Herein, the production of a useful protein includes obtaining a useful protein by transferring a gene encoding the useful protein as a target gene and by expressing the gene, and increasing the amount of the desired useful protein to be produced by controlling the specific gene expression with the expression of the target gene, and the like.

The plant to be used in the present invention is a plant capable of producing a useful protein. Therefore, the plant that can be used in the present invention may be a plant body modified so as to be able to produce a useful protein, for example, as a plant genetically modified by a gene recombination technique or the like so as to be able to produce a useful protein or a plant into which a virus vector modified so as to produce a useful protein has been transferred, may be a natural plant having a characteristic capable of producing a useful protein, or may also be a cultivar or the like that has been bred to have such a characteristic.

According to a preferred embodiment of the present invention, the plant is a plant modified so as to be able to produce a useful protein, and a stable genetically modified plant in a particularly preferred embodiment.

The plant species that can be used in the present invention is not particularly limited as long as it can produce a useful protein. Desirably, the plant species is a plant that can be transformed by an Agrobacterium method or a PEG method, and is a plant that stably expresses a target gene. For example, a dicot plant and a monocot plant can be mentioned. Specific examples of the dicot plant include a solanaceous plant (such as potato, tobacco, Nicotiana benthamiana, or tomato), a cruciferous plant (such as arugula, Brassica chinensis komatsuna, mizuna greens, Brassica juncea, or Arabidopsis thaliana), an asteraceae plant (such as lettuce, or artichoke), a leguminous plant (such as alfalfa, or soybean), a chenopodiaceae plant (such as spinach, or sugar beet), a labiatae plant (such as perilla, or basil), an umbelliferous plant (such as carrot, or Cryptotaenia japonica), a cucurbitaceae plant (such as melon, watermelon, cucumber, or pumpkin), and a malvaceae plant (such as cotton). Further, examples of the monocot plant include a gramineous plant (such as rice, wheat, barley, or maize), an araceae plant (such as taro, or konjac), and a liliaceae plant (such as onion, green onion, or asparagus). In addition, as the plant used for transformation, it is not necessarily required to be a wild-type plant, and a genetically modified plant can also be used. For example, in a case where glycoprotein is assumed as the useful protein, a genetically modified plant that expresses a human-type glycosyltransferase, or the like can also be used for the transformation.

According to a preferred embodiment of the present invention, the plant species to be used is any one selected from the group consisting of potato, lettuce, tobacco, tomato, rice, barley, wheat, soybean, and maize, and more preferably any one selected from the group consisting of potato, lettuce, tobacco, and rice.

In addition, in the present invention, the expression "plant body" means an organ, a portion, and a part of a plant, and includes, for example, a leaf, a stem, a root (including an adventitious root), a shoot (including an adventitious shoot), a petal, a seed leaf, an embryonic axis, an anther, an embryo (including an adventitious embryo), a seed, a fruit, an underground stem, a tuberous root, a tuber, a bulb, and a part thereof. At this time, typically, the expression "plant body" is used in the meaning of not including a modification site where the branching or extending is conducted even if the site is separated from a plant body (for example, a hairy root in hairy root culture). In other words, the expression "plant body" can typically be a plant body excluding a hairy root (or excluding only the hairy root). Preferably, the expression "plant body" means dedifferentiated cells, a cell cluster (callus), and an organ, a tissue, a portion and/or a part of a differentiated plant.

In the present invention, a plant body is cultured while keeping at least a part of the plant body immersed in a liquid medium.

The expression "at least a part" referred to herein includes the entire plant body, and further also includes, in addition to a case of being any one of the above-described organ, portion and part of a plant, or being a combination of the organ, portion and part of a plant, a case of being a part of the organ, portion and part of a plant.

Herein, the expression "liquid medium" is referred to as a liquid containing nutrients such as a carbon source and a nitrogen source which are essential for the growth of a plant and having an osmotic pressure and a pH which have been appropriately adjusted, for the purpose of favorably growing the plant, and may be a liquid basal medium known in a plant tissue culture technique, a modified culture medium of the liquid basal medium, or a culture medium obtained by removing some components from the liquid basal medium. As an example of such a known medium, a basal medium such as White medium (described on pp. 20 to 36 of "Shokubutsu Saibo Kogaku Nyumon (Introduction to plant cell engineering)" published by Gakkai Shuppan Center), Heller medium (Heller R, Bot. Biol. Veg. Paris 14 1-223 (1953)), SH medium (medium of Schenk and Hildebrandt), MS medium (medium of Murashige and Skoog, described on pp. 20 to 36 of "Shokubutsu Saibo Kogaku Nyumon (Introduction to plant cell engineering)" published by Gakkai Shuppan Center), LB medium (medium of Linsmaier and Skoog, described on pp. 20 to 36 of "Shokubutsu Saibo Kogaku Nyumon (Introduction to plant cell engineering)" published by Gakkai Shuppan Center), Gamborg medium, B5 medium (described on pp. 20 to 36 of "Shokubutsu Saibo Kogaku Nyumon (Introduction to plant cell engineering)" published by Gakkai Shuppan Center), MB medium, and WP medium (Woody Plant: for woody plants)can be mentioned.

The pH of the liquid medium can be appropriately adjusted according to the plant, and is preferably 5.0 to 6.5.

The amount of the liquid medium to be used, may be an amount with which a state in which at least a part of a plant body is immersed in the liquid medium is continuously kept throughout the culture period, and can be appropriately adjusted according to the size of a container, and as described in the later Examples, it is advantageous for the production of a useful protein when the plant body is immersed in a liquid, and therefore, the larger the amount of the liquid medium is, the more preferable it is. For example, it is preferred to use the liquid medium in an amount such that 50% or more, and more preferably 75% or more of the entire plant body is immersed in a liquid throughout the culture period.

In the present invention, in a case where the plant body to be used is a plant body that has been genetically modified so as to be able to produce a useful protein, the plant body is preferably a plant body obtained by transferring a target gene. In general, the target gene is transferred into a plant body as an expressible nucleic acid construct containing the target gene.

In the present invention, the expression "target gene" means a gene to be transferred into a plant or the part thereof. The target gene is not particularly limited as long as it can change a genetic trait of the plant as a result of being transferred into the plant, and may be a gene derived from an organism other than the plant to be transferred, and may also be an artificially produced gene. The artificially produced gene may be, for example, a chimeric gene in which two or more kinds of genes are linked together, or a mutated gene obtained by mutating a gene possessed by any organism. The mutated gene may be, for example, a gene obtained by deleting a part of the bases in the base sequence of DNA constituting a gene, or a gene obtained by performing the replacement of a part of the bases in the base sequence of DNA constituting a gene. Further, the mutated gene may be a gene obtained by inserting a partial base sequence in the base sequence.

In order to overexpress a target gene in a plant, it is only required to functionally link the gene to the downstream of an appropriate promoter, and to transfer the obtained nucleic acid construct into a plant cell by a known technique. According to a preferred embodiment of the present invention, a constitutive expression promoter can be used. Examples of the constitutive expression promoter include a cauliflower mosaic virus (CaMV) 35S promoter, an actin promoter or Elongation factor 1β promoter of rice, and a maize ubiquitin promoter, but are not limited to thereto, and the constitutive expression promoter may be selected and used according to the target tissue. In addition, the target gene can be overexpressed, and the useful protein can be accumulated in a specific organelle or secreted extracellularly, and further, for that purpose, an appropriate signal peptide can be provided.

Further, the target gene is incorporated into a vector together with suitably a marker gene, and optionally a reporter gene. As the marker gene, a drug resistance gene such as a kanamycin resistance gene (nptII), a hygromycin resistance gene (hptI), or a bleomycin resistance gene can be mentioned. As the reporter gene for confirming the expression position in a plant body, a luciferase gene, a β-glucuronidase (GUS), a green fluorescent protein (GFP), a red fluorescent protein (RFP), or the like can be mentioned. Therefore, the expressible nucleic acid construct of the present invention may contain a target gene together with a promoter, a marker gene, a reporter gene, and the like.

As the technique for transferring an expressible nucleic acid construct containing a target gene into a plant cell, for example, an Agrobacterium method, a particle gun method, a whisker method, a floral dip method, or the like can be mentioned.

The transfer technique will be described below by using an Agrobacterium method as an example.

In the Agrobacterium method, a plant is inoculated with an Agrobacterium bacterium that includes an expressible nucleic acid construct containing a target gene. The preparation method of the Agrobacterium bacterium used for the inoculation is as follows.

The usable Agrobacterium is not particularly limited as long as it is a microorganism capable of transferring a nucleic acid construct contained therein into a plant cell. As the usable Agrobacterium, for example, Agrobacterium tumefaciens (or Rhizobium radiobacter), or Agrobacterium rhizogenes (or Rhizobium rhizogenes) can be mentioned, and preferably, Agrobacterium tumefaciens can be mentioned.

Agrobacterium that includes an expressible nucleic acid construct containing a target gene can be produced by using any conventionally known technique.

For example, a target gene recombination intermediate vector in which a target gene or the like is incorporated into a plasmid capable of performing homologous recombination with a T-DNA region of a Ti plasmid possessed by Agrobacterium is prepared, and the target gene recombination intermediate vector may be transferred into Agrobacterium. Further, a target gene binary vector obtained by incorporating a target gene or the like into a binary vector that is generally used in an Agrobacterium method may be transferred into Agrobacterium.

That is, the expressible nucleic acid construct referred to in the present invention is a construct including a nucleic acid, which is designed so as to be able to express a target gene in an Agrobacterium-infected plant, and for example, as such a nucleic acid construct, a vector, a plasmid or the like, which contains a target gene, is mentioned.

Specific examples of the binary vector include a pBI-based vector (for example, pRiceFOX), a pPZP-based vector (Plant Molecular Biology 25(6): 989-94. (1994)), a pCAMBIA-based vector (vector skeleton: pPZP vector), and a pSMA-based vector (Plant Cell Reports 19: 448-453. (2000)), but are not limited to them.

In the present invention, as the plant body modified so as to be able to produce a useful protein, a plant body of a stable genetically modified plant, and further a plant body in which a virus vector is transferred can be mentioned, and a genetically modified plant is preferably mentioned, that is, a stable genetically modified plant is mentioned.

In this regard, the expression "stable genetically modified plant" is referred to as a stable transformant, and is referred to as a plant in which a gene transferred by passaging a plant body obtained in accordance with the above-described gene transfer technique of a genetically modified plant with a certain passage number and repeating the selection by using a predetermined marker or the like as a marker is stably maintained in the entire plant body.

The target gene in the present invention is a gene from which a protein as a useful substance can be obtained by the expression of the gene, and as such a useful protein, a protein for medical use, a protein for industrial use, or the like can be mentioned.

The protein for medical use is classified into a protein for therapeutic purposes, and a protein for diagnostic purposes, and as the protein for therapeutic purposes, a peptide, a vaccine, an antibody, an enzyme, a hormone (preferably, a peptide hormone), or the like can be mentioned. Specific examples of the protein for therapeutic purposes include a viral protein for use as a vaccine, a hematopoietic growth factor such as a granulocyte colony-stimulating factor (G-CSF), a granulocyte macrophage colony-stimulating factor (GM-CSF), erythropoietin (EPO), or thrombopoietin, a growth factor such as an epidermal growth factor (EGF), an insulin-like growth factor (IGF), a transforming growth factor (TGF), a nerve growth factor (NGF), a basic fibroblast growth factor (bFGF), or a platelet-derived growth factor (PDGF), a cytokine such as interferon, or interleukin (IL)-1 or IL-6, a monoclonal antibody or the fragment thereof, a tissue plasminogen activator (TPA), urokinase, serum albumin, a blood coagulation factor VIII, leptin, insulin, and a stem cell growth factor (SCF). In this regard, the expression "for therapeutic purposes" is used in the meaning of including also "for prophylactic purposes". Further, examples of the protein for diagnostic purposes include an antibody, an enzyme, and a hormone.

As the virus protein used as a vaccine, preferably, a component protein of a virus-like particle (VLP) can be mentioned. The component protein of a VLP may be a single protein or may contain one or more proteins. Examples of the virus include virus an influenza virus, a human immunodeficiency virus (HIV), a human hepatitis C virus (HCV), and a human hepatitis B virus (HBV). As the component protein of a VLP of an influenza virus, an influenza hemagglutinin (HA) protein, or the like can be mentioned.

The protein for industrial use is a protein used for food, feed, cosmetics, fibers, detergent, chemicals, or the like, and includes a peptide, an enzyme, and a functional protein. Specific examples of the protein for industrial use include a protease, a lipase, a cellulase, an amylase, an albumin, a peptidase, a luciferase, a lactamase, a collagen, a gelatin, a lactoferrin, a transferrin, a jellyfish green fluorescent protein (GFP), and a β-glucuronidase (GUS).

In the present invention, a plant body is cultured while keeping at least a part of the plant body immersed in a liquid medium to allow the plant body to grow. That is, in the present invention, when an immersed plant body is cultured, the plant body is continuously immersed in a culture medium without intermittently repeating the contact with a culture medium.

### Environmental conditions for performing the method according to the present invention

The method according to the present invention is desirably performed in an environment in which liquid culture of a plant body can be continuously performed.

Therefore, the liquid medium of the plant body is preferably in a sterile state or in an environment similar to the sterile state.

The conditions such as temperature, humidity, carbon dioxide concentration, light irradiation, and the like in a case where the method according to the present invention is performed are not particularly limited as long as the conditions are suitable for the growth of the plant body and the plant body can be grown.

For example, the temperature condition is, although depending on the kind of the plant or the part to be used, usually 10°C or more and preferably 20°C or more, and is usually 40°C or less and preferably 30°C or less.

Further, the humidity condition is preferably 50% or more, and is usually 100% or less.

The concentration condition of carbon dioxide can be usually 300 ppm or more and preferably 500 ppm or more, and can be usually 5000 ppm or less and preferably 3000 ppm or less.

As the light irradiation condition, a dark place or a bright place can be appropriately selected. As the light source used under the bright condition, it is not particularly limited, and sunlight, a fluorescent lamp, a light-emitting diode (LED), a cold cathode fluorescent lamp (CCFL, hybrid electrode fluorescent lamp (HEFL)), inorganic and organic electroluminescence (EL), or the like can be mentioned.

The light intensity can be evaluated by measuring the photosynthetic photon flux density (PPFD) or the like. The PPFD is expressed by the number of photons per unit time and per unit area of the light in a visible region of 400 to 700 nm effective for the photosynthesis, and the unit is µmol.m⁻²·s⁻¹. The PPFD is usually 2000 µmol.m⁻²·s⁻¹ or less, preferably 1500 µmol.m⁻²·s⁻¹ or less, and more preferably 1000 µmol.m⁻²·s⁻¹ or less for performing the measurement. In this regard, the PPFD can be measured by using a photon meter or the like.

The growth time of a plant body is usually 30 to 90 days, however, it differs depending on the plant species, the amounts of the plant body and a liquid medium to be charged, other environmental conditions, and the like, and can be appropriately adjusted in consideration of the growth amount of the plant body, the presence or absence of aging, or the like in a container.

### Extraction/separation step

The present invention can further include a step of extracting or separating a desired useful protein from the grown plant body or the liquid medium, from the obtained plant body. That is, it is preferred to obtain a fraction containing a desired protein from the grown plant body or the liquid medium, and to separate and purify the desired protein by an appropriate method. In this regard, in the nucleic acid construct containing a target gene encoding the desired protein, for example, a tag sequence for purification, and a signal sequence for secretion to the outside of the plant cell may be contained.

As the extract for use in a case where protein extraction is performed, a known buffer solution, for example, a glycine buffer solution, a citrate buffer solution, an acetate buffer solution, a succinate buffer solution, a malate buffer solution, a MES buffer solution, a PIPES buffer solution, a MOPS buffer solution, a phosphate buffer solution, a TES buffer solution, a Hepes buffer solution, a tricine buffer solution, a Tris buffer solution, a bicine buffer solution, a glycylglycine buffer solution, a TAPS buffer solution, a borate buffer solution, or the like can be used. When protein extraction is performed, a known protease inhibitor, for example, benzamide, phenylmethylsulfonyl fluoride (PMSF), 4-(2-Aminoethyl) benzenesulfonyl fluoride hydrochloride (AEBSF), antipain, chymostatin, leupeptin, pepstatin A, phsphramidon, aprotinin, ethylenediaminetetraacetic acid (EDTA), or the like can be used. When protein extraction is performed, a known antioxidant can be used, and for example, dithiothreitol (DTT), glutathione (GSH), β-mercaptoethanol, tris(2-carboxyethyl)phosphine (TCEP), cysteine, mercaptoethylamine, mercaptopropionic acid, or the like can be mentioned. Further, as the surfactant, a known surfactant can be used, and for example, TritonX-100, NP-40, Tween 20, CHAPS, CTAB, sodium cholate, sodium deoxycholate, sodium dodecyl sulfate (SDS)or the like can be mentioned. As the method for crushing a plant for protein extraction, a known method can be used, and for example, crushing by a bead-type crushing device, a mixer, an ultrasonic crusher, or the like can be performed.

In the present invention, the purification can be performed by a known technique. For example, as the known technique, affinity chromatography, ion exchange chromatography, gel filtration chromatography, hydrophobic interaction chromatography, immobilized metal affinity chromatography, reverse phase chromatography, hydroxyapatite chromatography, or the like can be mentioned, and these may be used in appropriate combination.

In a case where the protein is used for the application of an oral ingestion-type vaccine or the like, the obtained plant body can used by being processed as it is, for example, dried and powdered.

### Culture container

The method according to the present invention is performed in a container. When the method is performed in a container, the sterile state or the environment similar to the sterile state is easily maintained. That is, the expression "culture container" referred to herein is used in the meaning of not including a culture chamber itself in which a culture tank is arranged in a room, a greenhouse itself in which hydroponic cultivation can be performed, and the like. Preferably, it is favorable to perform the culture in a sterilized culture container, and more preferably, it is desirable to perform the culture in a container having an introduction part for introducing a gas such as air sterilized by a filter or the like and a discharge part.

Preferably, it is favorable to perform the method according to the present invention in a bag-type culture tank.

In addition, as the method according to the present invention, preferably, a container (or culture tank) for liquid immersion culture is mentioned. As such a container for liquid immersion culture, a culture tank that is made of a material that can be sterilized by steam or gamma rays, for example, polystyrene, polycarbonate, polyethylene terephthalate, or the like and is capable of having various forms of bag-type, box-type and the like can be accepted, but the container is not limited to such a culture tank.

In the present invention, as such a culture tank, a large-sized culture tank exceeding 100 to several hundreds of liters may be used, but by utilizing a small-sized culture tank with less than 20 liters in place of the large-sized culture tank and by preparing a large number of the small-sized culture tanks as needed, the culture is preferably performed.

As such a small-sized culture tank, a bag-type culture tank (culture bag) configured so as to be able to be self-stood by being expanded from the folded state is preferred. As a preferred example of such a culture tank, a bag-type culture tank as described below can be mentioned. That is, the method according to the present invention is preferably performed in such a bag-type culture tank.

### Example of bag-type culture tank

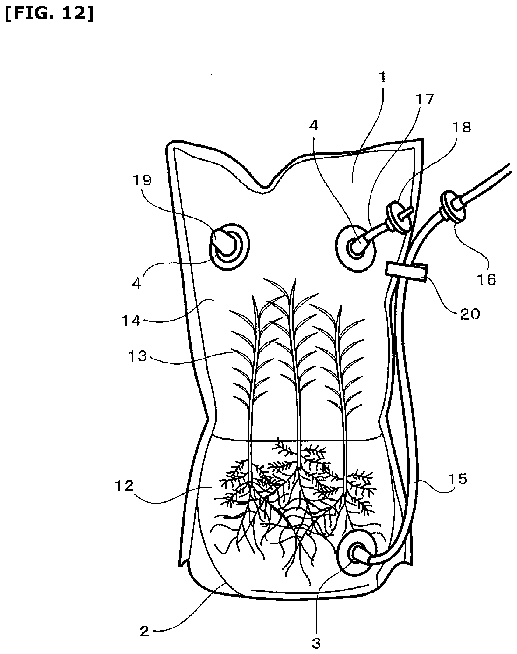

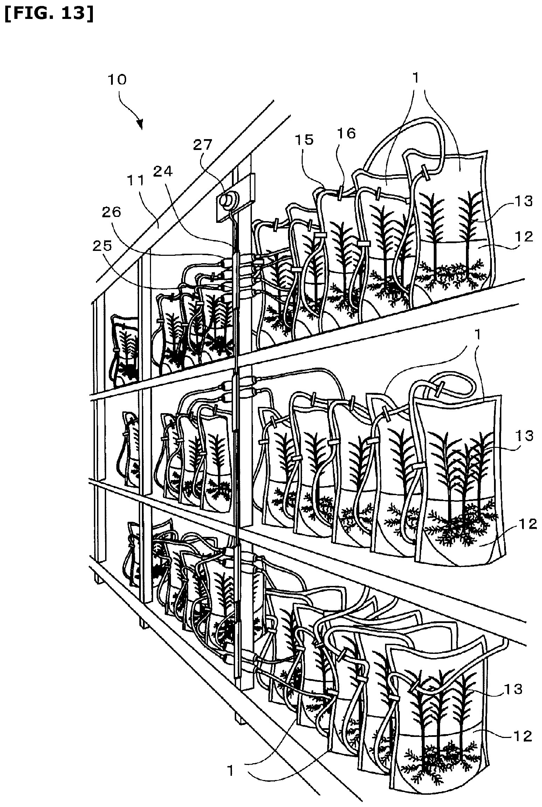

Examples of a preferred bag-type culture tank (culture bag) and a mass production facility using such a culture tank are illustrated in Figs. 12 and 13, respectively.

In Figs. 12 and 13, a culture bag 1 is prepared by using a composite film (having a thickness of 100 µm as an example) of polyethylene terephthalate (PET) and axially non-oriented polypropylene (CPP) as a material as an example of a soft resin, and by forming the film in a bag shape of a bottom gusset-type bag that is vertically long and provided with gussets 2 at the bottom. As the resin used for the material of the culture bag 1, a resin having transparency in the wavelength range of the light required for plant culture is selected. The upper end of the culture bag 1 is an opening part over the entire width, and the periphery of the culture bag 1 excluding the opening part is sealed. By expanding the culture bag 1 from the state in which gussets 2 are folded, the bottom of the culture bag 1 is expanded to a certain size. As a result, the self-standing property is generated on the culture bag 1, and the culture bags 1 can be arranged side by side without using a support means. Further, by folding a gusset 2 when the gusset 2 is not used, a large number of culture bags 1 can be stored in a relatively narrow space.

A single lower port 3 and two upper ports 4 are attached to the side of the culture bag 1. These ports 3 and 4 are for introducing a gas to the inside of the culture bag 1, or for exhausting a gas from the culture bag 1. The ports 3 and 4 are provided by adhering molded articles that have been made by using, for example, a polypropylene resin as a raw material to the culture bag 1, respectively. The ports 3 and 4 are formed, for example, in a shape capable of connecting tubes. In addition, the area from the upper end of the upper ports 4 to the upper end of the culture bag 1 functions as a sealing part for sealing the culture bag 1 after a culture medium and a plant material are housed in the culture bag 1.

The materials for a culture bag 1 and ports 3 and 4 can be appropriately changed as long as the materials can withstand autoclave treatment for sterilizing the culture bag 1. The capacity of the culture bag 1 may be appropriately set as long as the capacity is within the range where the worker can lift a single culture bag 1 in which a culture medium and a plant material have been housed. In the present embodiment, the capacity of a culture bag 1 is set to at most 12 liters (L), and preferably 8 L, and the volume of the total of the culture medium and the immersed plant material to be housed in the culture bag 1 is set to around 60% of the capacity of the culture bag 1, that is, the volume of the total of the culture medium and the immersed plant material is set to 7 L as a guide in a case where the capacity of the culture bag 1 is 12 L, and is set to 5 L as a guide in a case where the capacity of the culture bag 1 is 8 L.

Fig. 13 shows an example of the use of culture bags 1 in the production process of plants, which are an example of a mass production facility. Fig. 12 shows an example of a single culture bag 1 in the use state. As shown in Fig. 12, in the present embodiment, a storage rack 11 is arranged in a predetermined culture chamber 10. The culture chamber 10 is sectioned as a culture space in which various physical parameters constituting a plant culture environment such as temperature, humidity, and lighting can be controlled.

A large number of culture bags 1 are arranged side by side on each shelf of the storage rack 11. As shown in detail in Fig. 12, in each of the culture bags 1, a culture medium 12 and a plant material 13, which have been sterilized by filter filtration or the like, are housed, and the opening part at the upper end of each of the culture bags 1 is sealed in a sterile state by using a sealing method such as heat sealing. On the upper side of the culture medium 12, a gas phase part 14 is secured. As the culture medium 12, a liquid medium having an adequate composition is appropriately selected and used according to the type of the plant material 13 and the production process thereof. A vent tube 15 and a filter 16 are connected to a lower port 3 of the culture bag 1, a vent tube 17 and a filter 18 are connected also to one of upper ports 4, and a filter 19 is connected to the other of the upper ports 4. A predetermined gas, for example, air or carbon dioxide is introduced into the culture bag 1 from the vent tubes 15 and 17, and in exchange for the introduction, the gas in the culture bag 1 is exhausted from the filter 19. In this regard, each of the upper ports 4 may be used for the exhaust. Further, it is desirable that the arrangement position of the lower port 3 from the bottom of the culture bag 1 and the amount of the culture medium can be adjusted so that a predetermined gas is aerated from the lower port 3 into the liquid medium. By the aeration into the liquid medium, a predetermined gas is efficiently supplied to a plant body, and further a stirring effect of the liquid medium or the plant body can also be expected. The gas aeration rate may be appropriately selected according to the type of a plant or the growth method, and is preferably 50 to 100 mL/min. The vent tubes 15 and 17 are fixed at appropriate positions on the culture bag 1 by using clips 20 or the like in order to prevent the entanglement and the like of the tubes.

In addition, as described above, the above-described bag-type culture tank and a facility using the bag-type culture tank are both examples of a culture tank and a culture chamber, respectively, which are environments for performing the method according to the present invention.

That is, according to a preferred embodiment of the present invention, the method according to the present invention can be a method for mass-producing a useful protein by culturing a large amount of plants by using multiple containers.

### Examples

Hereinafter, the present invention will be described in detail by way of the following Examples, however, the present invention is not limited to the Examples.

### (Example 1) Comparison of growth amounts of plant and production amounts of useful protein in solid medium and liquid medium

In order to compare the growth rate by the culture method for a plant body, the amounts of growth of the potato plant bodies by a solid culture method and by a liquid culture method were compared with each other in a plant box.

### Plant material and the transformation:

As a plant, tissue culture seedlings of a variety of potato (Solanum tuberosum L.) "Sassy" were used.

As the transformation method, an Agrobacterium method was employed. In the Agrobacterium method, one in which a β-glucuronidase gene (GUS gene) was incorporated onto the downstream side of a cauliflower mosaic virus 35S promoter as a vector for plant transformation and which had a kanamycin resistance gene as a selection marker gene was transferred in Agrobacterium tumefaciens LBA4404 (product code 9115, obtained from TAKARA BIO INC.) being an Agrobacterium strain by an electroporation method (for example, Gene Pulser II, manufactured by Bio-Rad Laboratories, Inc. under the pulse condition of 25 µF, 200 Ω, and 2.25 kV), and was used.

The transformation of potato was performed in accordance with the description in the Document [T. Momma, Shokubutsu Soshiki Baiyo (Plant tissue culture) Vol. 7: 57-63 (1990)].

A microtuber obtained from a variety of potato "Sassy" (Japan Potato) was sliced into pieces each having a thickness of 2 to 3 mm, and the sliced pieces were taken as materials for Agrobacterium infection. The sliced pieces were immersed in the above-described Agrobacterium bacterial liquid, and then the immersed pieces were placed on sterile filter paper to remove the excess Agrobacterium. The resultant pieces were placed on a MS medium (containing 1 ppm of Zeatin, 0.1 ppm of indole-3-acetic acid (IAA), 100 µM of acetosyringone, and 0.8% of agar) in a Petri dish, and the culture was performed at 25°C for 3 days under the conditions of lighting for 16 hours (with a photon flux density of 32 µE/m2s) / non-lighting for 8 hours.

Next, the resultant pieces were cultured for one week in a culture medium containing 250 ppm of carbenicillin in place of the acetosyringone. After that, the cultured pieces were transferred onto a culture medium containing 50 ppm of kanamycin being a selection marker antibiotic, and passaged every two weeks. During this time, an adventitious shoot was formed and a shoot was generated.

The grown shoots were placed on a MS medium containing 250 ppm of carbenicillin and 100 ppm of kanamycin but not containing any plant growth regulating substance. The rooted shoots were subjected to polymerase chain reaction (PCR, under the conditions of 95°C for 5 minutes, 30 times of (95°C for 30 seconds, 55°C for 30 seconds, and 72°C for 1 minute), and 72°C for 10 minutes) so that the individuals containing a kanamycin resistance gene as an exogenous gene were detected from among the kanamycin-resistant grown plant bodies. It was thus confirmed that the redifferentiated plant body was a transgenic plant body.

In this regard, as a primer for specifically amplifying the sequence of the kanamycin resistance gene, TAAAGCACGAGGAAGCGGT (SEQ ID NO: 1) and GCACAACAGACAATCGGCT (SEQ ID NO: 2) were used.

As described above, 3 lines of the transgenic plant bodies of potato into which the β-glucuronidase gene had been transferred were obtained. In experiment, pKT19-1 was used as a representative line among the obtained 3 lines.

### Culture method:

The in vitro culture conditions for potato were as follows.

As the culture container, a plant box (obtained from SANSYO Co., LTD) was used. The obtained transgenic plant body was passaged by using an apical bud in a solid medium. As the solid medium, a culture medium in which 0.8% of agar had been added into a MS medium (containing 3% of sucrose and having a pH of 5.8) was used.

In a case of a liquid medium, a MS medium (containing 3% of sucrose and having a pH of 5.8) was used. In a case of growing a plant body in a bag-type culture tank, a liquid medium, which had been prepared by adding 20 g/L of sucrose and 0.02 ppm of benzylaminopurine into a modified MS medium (modified to have 3 times amount of potassium dihydrogen phosphate and 4 times amount of calcium chloride) and by adjusting the pH to 5.8, was used.

### Verification method of production amount of useful protein:

As an index showing the production amount of β-glucuronidase being a useful protein, the β-glucuronidase(GUS) activity was measured as follows.

Around 30 mg fresh weight of a sample and 1 ml of an extraction buffer were placed in a 2-ml tube, and crushed at 4°C with Mixer Mill MM300 (manufactured by Retsch GmbH). The composition of the extraction buffer was a 25 mM phosphate buffer (pH 7.0), 10 mM EDTA, 10 mM DTT, and 0.1% Triton X-100. After the crushing, the content in the tube was centrifuged (at 15,000 rpm and 4°C for 10 minutes) to obtain a supernatant as a protein extract. Alternatively, an extraction buffer in an amount of 1 ml per 1 g of fresh weight of a sample was added to the sample, the sample with the extraction buffer was subjected to crushing with a pestle and a mortar to obtain a crush liquid, the crush liquid was filtered with gauze, and the obtained filtrate was subjected to centrifugation (at 3000 rpm and 4°C for 20 minutes) to obtain a supernatant as a protein extract.

The protein concentration of the protein extract was quantified by a calibration curve obtained by using bovine serum albumin and using a RC DC protein assay kit II (manufactured by Bio-Rad Laboratories, Inc).

The measurement of the GUS activity was performed by adding 2 µl of a crude enzyme solution and 50 µl of 5 mM 4-methylumbelliferyl-β-D-glucuronide as a substrate into 150 µl of an extraction buffer as a reaction mixture, and by measuring the generated fluorescence.

### Results:

The results were as follows.

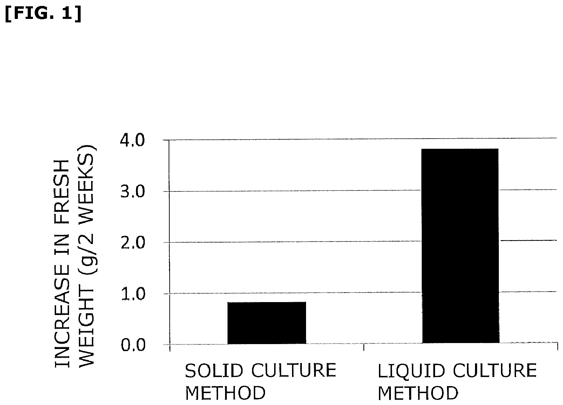

When comparing the increase in the fresh weight after 2 weeks of culture, 0.8 g for the solid culture method and 3.8 g for the liquid culture method were obtained, and thus the increase in the liquid culture method was 4.8 times larger than that in the solid culture method. From the data, it was indicated that the liquid culture method is more suitable than the solid culture method for the growth of a plant body (Fig. 1).

### (Example 2) Comparison of growth amounts of plant and production amounts of useful protein by liquid medium amount

Next, the amounts of growth with different amounts of a liquid medium were compared with each other.

The plant material used, the verification method of the production amount of a useful protein, and the composition of the liquid medium were similar to those in Example 1.

### Culture method of plant:

In a bag-type culture tank (with a 8-L volume), around 2 g of a plant body that had been cultured for 2 months in a plant box by using a solid medium was placed in each of 1 L of a liquid medium (1-L section) and 4 L of a liquid medium (4-L section), and the growth of the genetically modified potatoes expressing the GUS was compared after 40 days of culture.

### Results:

The results were as follows.

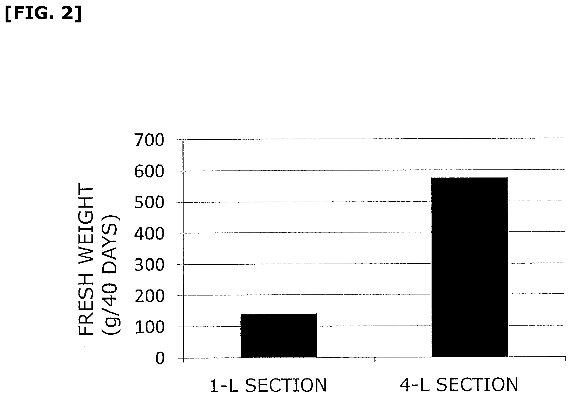

When comparing the fresh weight after 40 days of culture, 139 g for the 1-L section and 576 g for the 4-L section were obtained, and thus the yield amount in the 4-L section was 4.1 times larger than that in 1-L section (Fig. 2). At this time, the growth in each of the 1-L section and the 4-L section was favorable, and in the 1-L section, the growth into the upper part (gas phase part) from the liquid surface of the liquid medium was observed, and in the 4-L section, the growth into the liquid medium (liquid phase part) was mainly observed.

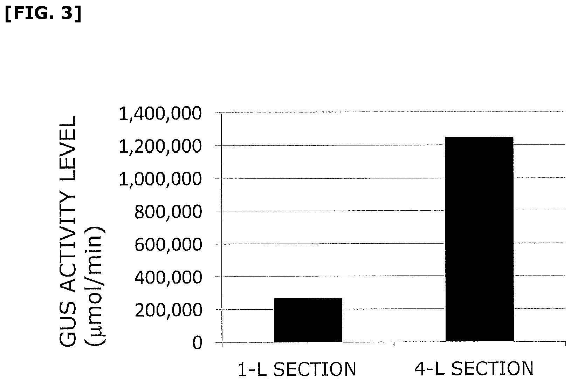

At this time, the amount of the GUS produced per bag-type culture tank was 269521 µmol/min for the 1-L section and 1248768 µmol/min for the 4-L section, and thus the production amount in the 4-L section was 5.0 times larger than that in the 1-L section (Fig. 3).

The culture method in which a plant body was immersed in a liquid at all times was generally avoided as a method for growing plant seedlings because the vitrification of the tissue was generated and the acclimation after growth was difficult. However, in the production of a useful protein, the robustness in consideration of the acclimation process was not required, which was different from the growth of plant seedlings, and therefore, it was indicated that the liquid immersion culture of a plant body was possible and the yield amount per unit culture volume was increased.

### (Example 3) Comparison of useful protein contents to the total mass of protein in liquid immersion part and gas phase part of plant body

A genetically modified potato plant body expressing the same GUS as that in each of Examples 1 and 2 was subjected to liquid immersion culture for 40 days by using a bag-type culture tank, and the GUS activity for the total protein in each of a leaf and a stem was compared between the part immersed in a liquid medium all the time (liquid phase part) and the part grown into the upper part from the liquid surface(gas phase part). The conditions were set to be similar to those in Example 1 and 2.

### Results:

The results were as follows.

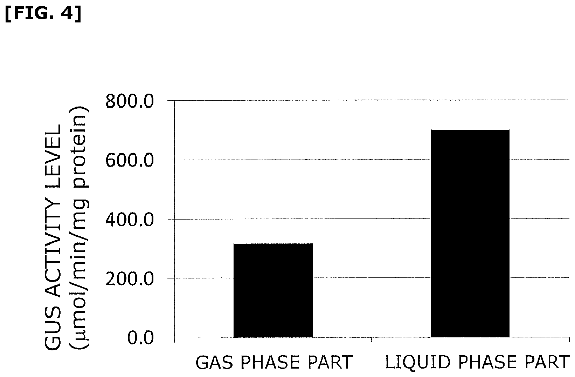

The GUS activity for the total protein was higher in the liquid phase part immersed in a liquid medium all the time than that in the gas phase part. That is, in the leaf, 315.8 µmol/min/mg protein for the gas phase part and 700.8 µmol/min/mg protein for the liquid phase part were obtained, and thus the GUS activity in the liquid phase part was 2.2 times higher than that in the gas phase part (Fig. 4).

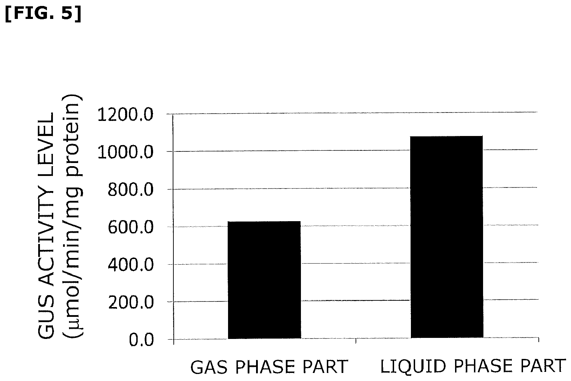

Further, in the stem, 626.3 µmol/min/mg protein for the gas phase part and 1075.1 µmol/min/mg protein for the liquid phase part were obtained, and thus the GUS activity in the liquid phase part was 1.7 times higher than that in the gas phase part (Fig. 5).

From these results, it was indicated that the culture performed in a state of being immersed in a liquid was suitable for the production of a useful protein.

### (Example 4) Comparison of production amounts of useful protein in liquid immersion culture and in soil cultivation

The GUS activity value in a leaf at the time of liquid immersion culture, which was confirmed in Example 3 above, was compared with that in a leaf in a case where the same genetically modified plant as that used in the liquid immersion culture was grown in soil cultivation. The soil cultivation conditions in a greenhouse of potato were as follows.

An apical bud of the obtained transgenic plant body was passaged in a solid medium, and the in vitro seedlings after the lapse of 14 weeks were acclimated to horticultural soil (Compal luxury horticultural soil, Sumirin Nosan Kogyo KK), and grown for 4 weeks in a greenhouse.

### Results:

The results were as follows.

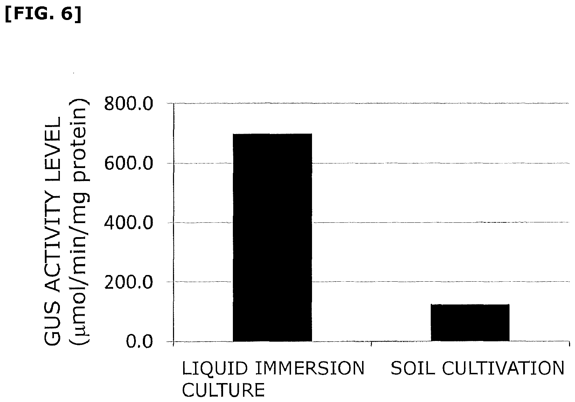

As compared with the GUS activity for the total protein in a leaf of the liquid immersion culture, the GUS activity for the total protein was lowered to 123.9 µmol/min/mg protein in a leaf of the soil cultivation (Fig. 6). From these things, it was able to be confirmed that the method for producing a useful protein by liquid immersion culture, which was achieved this time, was an industrially useful method.

### (Example 5) Comparison of production amounts of useful protein in solid culture and liquid immersion culture of leaf lettuce

In order to compare the amount of the useful protein produced by the culture method for a plant body in leaf lettuce, the production amounts obtained by the solid culture method and the liquid immersion culture method were compared with each other.

### Plant material and the transformation:

As a plant, tissue culture seedlings of a variety of leaf lettuce (Lactuca sativa) "Greenwave" were used.

As the transformation method, an Agrobacterium method was employed. In the Agrobacterium method, one in which a β-glucuronidase gene (GUS gene) or a transferrin gene was incorporated onto the downstream side of a cauliflower mosaic virus 35S promoter as a vector for plant transformation and which had a hygromycin resistance gene as a selection marker gene was transferred in Agrobacterium tumefaciens LBA4404 (product code 9115, obtained from TAKARA BIO INC.) being an Agrobacterium strain by an electroporation method (for example, Gene Pulser II, manufactured by Bio-Rad Laboratories, Inc. under the pulse condition of 25 µF, 200 Ω, and 2.25 kV), and was used.

The transformation of leaf lettuce was performed in accordance with the description in the literature [Transformation protocol (plant edition), edited by Yutaka Tabei, and published by Kagaku-Dojin Publishing Company, INC].

The green leaf obtained from a variety of leaf lettuce "Greenwave" was cut into pieces each having a size of around 5 mm × 5 mm with a surgical knife, and the cut pieces were taken as materials for Agrobacterium infection. The cut pieces were immersed in the above-described Agrobacterium bacterial liquid, and then the immersed pieces were placed on sterile filter paper to remove the excess Agrobacterium. The resultant pieces were placed on a MS medium (containing 1 ppm of benzyladenine (BA), 0.1 ppm of naphthalene acetic acid (NAA), 100 µM of acetosyringone, and 0.3% of Gelrite) in a Petri dish, and the culture was performed at 25°C for 3 days under the condition of a dark place.

Next, the cultured pieces were transferred onto a culture medium containing 250 ppm of carbenicillin being a bactericidal agent and 25 ppm of hygromycin being a selection marker antibiotic, and passaged every two weeks. During this time, an adventitious shoot was formed and a shoot was generated.

The grown shoots were placed on a MS medium containing 250 ppm of carbenicillin and 25 ppm of hygromycin but not containing any plant growth regulating substance. The rooted shoots (plant bodies) were subjected to PCR (under the conditions of 95°C for 3 minutes, 35 times of (95°C for 20 seconds, 62°C for 15 seconds, and 72°C for 30 seconds), and 72°C for 5 minutes) so that the individuals containing a hygromycin resistance gene were detected from among the rooted shoots (plant bodies). It was thus confirmed that the redifferentiated plant body was a transgenic plant body.

In this regard, as a primer for specifically amplifying the sequence of the hygromycin resistance gene, CGGAAGTGCTTGACATTGG (SEQ ID NO: 3) and AGAAGAAGATGTTGGCGACC (SEQ ID NO: 4) were used.

As described above, the transgenic plant bodies of leaf lettuce into which a β-glucuronidase gene or a transferrin gene had been transferred were obtained.

### Culture method:

The in vitro culture conditions for leaf lettuce were as follows.

As the culture container, a plant box (obtained from SANSYO Co., LTD) was used. The obtained transgenic plant body was passaged by using an apical bud in a solid medium. As the solid medium, a culture medium in which 0.8% of agar had been added into a MS medium (containing 3% of sucrose and having a pH of 5.8) was used.

In a case of a liquid medium, a MS medium (containing 3% of sucrose, 0.02 ppm of BA, and 0.001 ppm of gibberellin, and having a pH of 5.8) was used.

### Verification method of production amount of useful protein (GUS):

As an index showing the production amount of β-glucuronidase being a useful protein, the β-glucuronidase(GUS) activity was measured as follows.

Around 100 mg fresh weight of a sample and 300 µL of an extraction buffer were added into a 3-ml tube, and crushed with a Multi-beads shocker (manufactured by Yasui Kikai Corporation). The composition of the extraction buffer was 20 mM Tris-HCL, and 1% of TritonX-100. After the crushing, the content in the tube was centrifuged (at 15,000 rpm and 4°C for 10 minutes) to obtain a supernatant as a protein extract.

The protein concentration of the protein extract was quantified by a calibration curve obtained by using bovine serum albumin and using a TaKaRa Bradford Protein Assay Kit (obtained from TAKARA BIO INC).

The measurement of the GUS activity was performed in a similar manner as in Example 1. That is, the measurement of the GUS activity was performed by adding 2 µl of a crude enzyme solution and 50 µl of 5 mM 4-methylumbelliferyl-β-D-glucuronide as a substrate into 150 µl of an extraction buffer as a reaction mixture, and by measuring the generated fluorescence.

### Verification method of production amount of useful protein (transferrin):

The production amount of transferrin being a useful protein was measured as follows.

Around 100 mg fresh weight of a sample and 300 µL of an extraction buffer were added into a 3-ml tube, and crushed with a Multi-beads shocker (manufactured by Yasui Kikai Corporation). The composition of the extraction buffer was 20 mM Tris-HCL, and 1% of TritonX-100. After the crushing, the content in the tube was centrifuged (at 15,000 rpm and 4°C for 10 minutes) to obtain a supernatant as a protein extract.

The protein concentration of the protein extract was quantified by a calibration curve obtained by using bovine serum albumin and using a TaKaRa Bradford Protein Assay Kit (obtained from TAKARA BIO INC).

The content of the transferrin in an extract was measured by using an enzyme-linked immunosorbent assay (ELISA) available on the market (Human Transferrin ELISA Kit, Bethyl Laboratories Inc., E88-128). The extract was diluted 1,000 times with a buffer included in the kit, and the diluted extract was tested.

### Results:

The results were as follows.

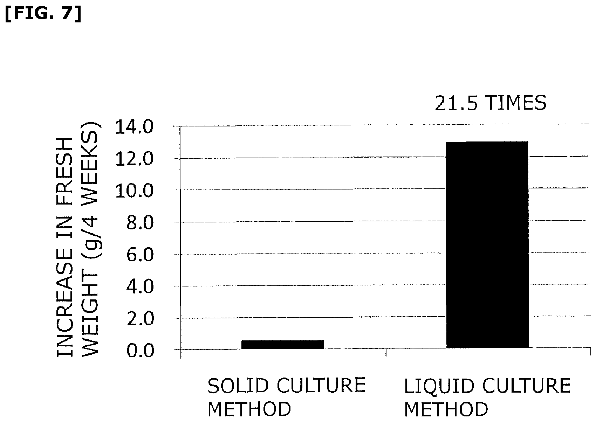

When comparing the increase in the fresh weight after 4 weeks of culture, 0.6 g for the solid culture method and 12.9 g for the liquid culture method were obtained, and thus the increase in the liquid culture method was 21.5 times larger than that in the solid culture method. From the data, it was indicated that the liquid culture method is more suitable than the solid culture method for the growth of a plant body (Fig. 7).

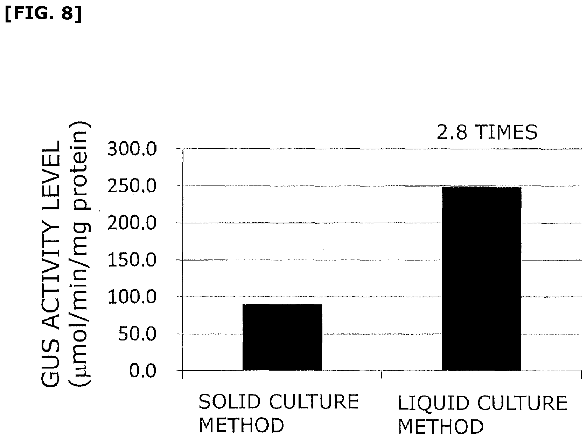

The GUS activity for the total protein was 90.0 µmol/min/mg protein in a leaf for the solid culture and 248.7 µmol/min/mg protein in a leaf for the liquid immersion culture, and thus the GUS activity in the liquid immersion culture was 2.8 times higher than that in the solid culture (Fig. 8).

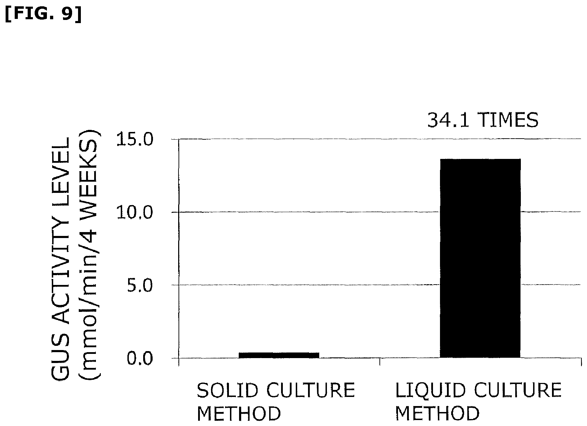

When comparing the amount of the GUS produced per culture container after 4 weeks of culture, 0.4 mmol/min for the solid culture method and 13.7 mmol/min for the liquid immersion culture method were obtained, and thus the increase in the liquid immersion culture method was 34.1 times larger than that in the solid culture method (Fig. 9).

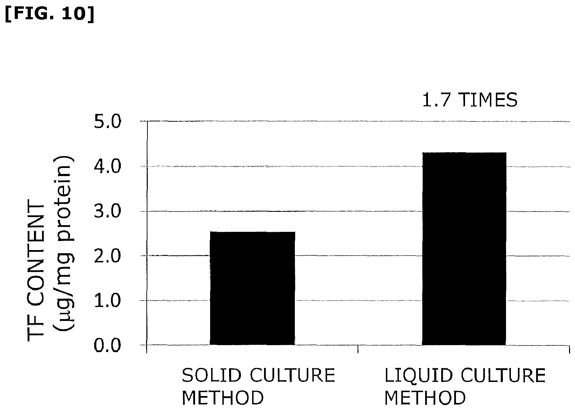

In addition, the content of transferrin for the total protein was 2.5 µg/mg protein in a leaf for the solid culture and 4.3 µg/mg protein in a leaf for the liquid immersion culture, and thus the content of transferrin in the liquid immersion culture was 1.7 times larger than that in the solid culture (Fig. 10).

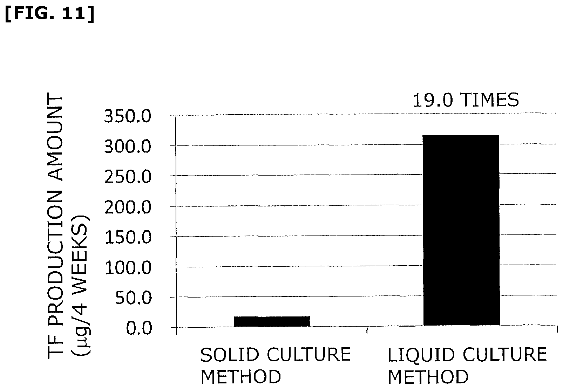

Further, when comparing the amount of the transferrin produced per culture container after 4 weeks of culture, 16.6 µg for the solid culture method and 315.1 µg for the liquid immersion culture method were obtained, and thus the increase in the liquid immersion culture method was 19.0 times larger than that in the solid culture method (Fig. 11).

The culture method in which a plant body was immersed in a liquid at all times was generally avoided as a method for growing plant seedlings because the vitrification of the tissue was generated and the acclimation after growth was difficult. However, in the production of a useful protein, the robustness in consideration of the acclimation process was not required, which was different from the growth of plant seedlings, and therefore, it was indicated that the liquid immersion culture of a plant body was possible and the yield amount per unit culture volume was increased.

### Reference Signs List

- 1: Culture bag
- 2: Gusset
- 3: Lower port
- 4: Upper port
- 10: Culture chamber (culture space)
- 11: Storage rack
- 12: Culture medium
- 13: Plant material
- 24: Branch pipe line
- 25: Manifold
- 26: Tube joint
- 27: Opening and closing valve

## Claims

1. A method for producing a useful protein by a plant, comprising:
culturing a plant body capable of producing a useful protein in a container while keeping at least a part of the plant body immersed in a liquid medium to allow the plant body to grow.

2. The method according to claim 1, wherein
the plant body is a plant body modified so as to be able to produce a useful protein.

3. The method according to claim 1 or 2, wherein
the plant body is a stable genetically modified plant.

4. The method according to any one of claims 1 to 3, wherein
the useful protein to be produced is a protein for medical use or a protein for industrial use.

5. The method according to any one of claims 1 to 4, wherein
the protein is a protein expressed by using a constitutive expression promoter.

6. The method according to any one of claims 1 to 5, further comprising a step of:
extracting or separating a useful protein from a grown plant body.

7. The method according to any one of claims 1 to 6, wherein
a plant species to be used is any one selected from the group consisting of potato, lettuce, tobacco, tomato, rice, barley, wheat, soybean, and maize.

8. The method according to any one of claims 1 to 7, wherein
culture and growth of the plant body are performed in a sterilized container.

9. The method according to any one of claims 1 to 8, wherein
the container is a bag-type culture tank.

10. The method according to any one of claims 1 to 9, wherein
a large amount of plants are cultured by using a plurality of containers to mass-produce the useful protein.


SEQUENCE LISTING

<110>  Kirin Company, Limited

<120>  Method for production of valuable materials using plants

<130>  800487PX01

<160>  4

<170>  PatentIn version 3.5

<210>  1
<211>  19
<212>  DNA
<213>  Artificial Sequence

<220>
<223>  primer

<400>  1
taaagcacga ggaagcggt                                                    19


<210>  2
<211>  19
<212>  DNA
<213>  Artificial Sequence

<220>
<223>  primer

<400>  2
gcacaacaga caatcggct                                                    19


<210>  3
<211>  19
<212>  DNA
<213>  Artificial Sequence

<220>
<223>  primer

<400>  3
cggaagtgct tgacattgg                                                    19


<210>  4
<211>  20
<212>  DNA
<213>  Artificial Sequence

<220>
<223>  primer

<400>  4
agaagaagat gttggcgacc                                                   20